(19) **Europäisches Patentamt**
**European Patent Office**
**Office européen des brevets**

(11) **EP 4 170 360 A1**

(12) **EUROPEAN PATENT APPLICATION**

(43) Date of publication:
**26.04.2023 Bulletin 2023/17**

(21) Application number: **21306476.9**

(22) Date of filing: **22.10.2021**

(51) International Patent Classification (IPC):
**G01R 29/08** $^{(2006.01)}$ **G09B 23/30** $^{(2006.01)}$
**H04B 17/00** $^{(2015.01)}$ **H04M 1/24** $^{(2006.01)}$

(52) Cooperative Patent Classification (CPC):
**G01R 29/0857; A61B 18/1815; A61N 5/02;**
A61B 2018/00071

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Université de Rennes 1**
  **35000 Rennes (FR)**
• **Centre National de la Recherche Scientifique
  CNRS**
  **75016 Paris (FR)**
• **Institut National des Sciences Appliquées de
  Rennes**
  **35708 Rennes (FR)**

• **Universite de Nantes**
  **44000 Nantes (FR)**
• **Centrale Supélec**
  **91190 Gif-sur-Yvette (FR)**

(72) Inventors:
• **ZHADOBOV, Maxim**
  **35700 RENNES (FR)**
• **BORYSKIN, Artem**
  **35235 THORIGNE FOUILLARD (FR)**
• **SAULEAU, Ronan**
  **35690 ACIGNE (FR)**

(74) Representative: **Plasseraud IP**
  **66, rue de la Chaussée d'Antin**
  **75440 Paris Cedex 09 (FR)**

(54) **PHANTOM DEVICE AND ELECTROMAGNETIC DOSIMETRY SYSTEM ASSOCIATED**

(57)     A phantom device (10) for reproducing at least one electromagnetic (EM) characteristic of a reference object made of an electromagnetic lossy medium, in particular a biological tissue, when illuminated by an EM wave with a predetermined frequency f1 emitted by an EM source (2), said phantom device comprising at least one unit structure, said unit structure comprising:
- at least one first dielectric layer (12) comprising an upper surface (13) face to the electromagnetic source (2) and a bottom surface (14) opposite to the upper surface (13),
- said upper surface (13) being at least partly transparent to the electromagnetic waves emitted by the source (2);
- said bottom surface (14) being at least partly reflecting for the electromagnetic waves transmitted through the first dielectric layer (12);
- said at least one first dielectric layer (12) characterized by a complex dielectric permittivity of its bulk material $\varepsilon_1^* = \varepsilon_1' - j\varepsilon_1''$ having an absolute value in a range between 3 and 40;
- said at least one first dielectric layer (12) further characterized by a thickness $T_1$ at least equal to 1/4 of the wavelength of the EM wave in the medium of the first dielectric layer (12).

FIG. 3

## Description

### Field of the invention

The invention relates to a phantom device for electromagnetic dosimetry, and more particularly, the present invention relates to a phantom device for simulating electromagnetic characteristics of a reference object made of a lossy medium, e.g., a biological tissue, particularly a human tissue.

### Background Art

With the development of the wireless technology, in particularly in case of cell phones which are used in proximity of the human body, precisely determining the electromagnetic exposure level in a human body becomes a critical factor.

Various categories of devices reproducing electromagnetic properties of biological tissues, commonly referred as "phantoms", for evaluating the exposure of the human body to electromagnetic waves, have been proposed.

To reproduce the electromagnetic response at the interface between the air and the surface of the skin, liquid phantoms have been proposed. Such phantom devices comprise a plastic part or a solid shell filled with a gel or a liquid having similar generic electromagnetic properties to those of the human biological tissues at the measurement frequency. These devices have several problems: the liquid needs to be changed frequently due to the evaporation and/or the degradation of its physical properties over time. These devices require special test equipment to support their weight and water content. Such devices are generally used in the frequency range from 300 MHz to 6 GHz and not suitable for electromagnetic dosimetry at frequencies above 6 GHz because of the shallow penetration depth of electromagnetic fields at these frequencies into the medium.

A solid phantom has been provided as an alternative solution. The solid phantom is a solid piece made for example of ceramic, graphite or carbon elements, or synthetic rubbers. The main advantage is their lifetime, in other words the constancy of the electromagnetic properties over time. However, these devices are quite complex to fabricate and costly to manufacture, generally requiring particularly high temperatures and high pressures. Furthermore, the solid phantoms, same as the liquid phantoms, designed to operate at frequencies below 6 GHz, cannot be used at higher frequencies due to the frequency-dependent variation of the material properties leading to high EM losses. Thus, the solid phantom can be used to overcome the evaporation problem intrinsic to the liquidbased phantoms. However, solid phantoms reproducing complex permittivity of biological tissues suffer from relatively high electromagnetic loss at frequencies above 6 GHz. For instance at 60 GHz, the penetration depth of electromagnetic radiation in the human tissues is of the order of 0.5 mm and therefore the absorption of the EM radiation is essentially limited to the superficial layers of the body. This leads to a prohibitively low signal-to-noise ratio (SNR) for any sensor embedded in a phantom reproducing the EM properties of said biological tissue.

The issue of the high electromagnetic loss in the upper part of the microwave spectrum is intrinsic to all soft biological tissues, characterized by a high imaginary part of the complex permittivity due to dominant water concentration, and constitutes an intrinsic constrain in the design of dosimetry devices. Thus, a phantom device made of a material, that reproduces the complex permittivity of such a lossy medium is not compatible with the compliance testing of wireless devices of 5G and beyond generations operating at frequencies above 6 GHz.

The document WO2017/0173350 describes a solid phantom device having characteristic of human tissues. It comprises one layer of a dielectric material doped with conductive microparticles of carbon, bearing a metalized shielding with a plurality of openings, and an array of sensors. The sensors are configured to measure the electromagnetic field emitted by an electromagnetic source and transmitted through the phantom. This solid phantom allows to overcome the evaporation problem intrinsic to the liquid phantom device, while the desired value of the reflection coefficient has been provided thanks to the conductive doping. However, the medium used in this proposed phantom device still suffers from significant losses of the electromagnetic field intensity during its propagation through the compound dielectric material at high frequencies, above 6 GHz.

Therefore, there is a need for a new and optimal design of phantom device for electromagnetic dosimetry that can be used to carry out measurements more precisely than previous solutions especially at frequencies above 6GHz.

It is an object of the present invention to provide a phantom device which can reproduce on demand the electromagnetic response from a surface of a biological tissue, for example a human tissue, which may represent a reference object made of an electromagnetic lossy material, at a predetermined frequency in a frequency range from 6 GHz to at least 300GHz, while enhancing the electromagnetic wave penetration depth, by using a phantom structure made of lower loss dielectric material. Such a new phantom device can be used in an electromagnetic dosimetry system to improve the signal to noise ratio.

Another aim of the present invention is to propose a phantom device which can be easily attached to or wrapped over a 3D shaped object, for example reproducing the anatomical form of a part of the human body.

Another aim of the present invention is to propose a phantom device in a shape different from the shape of the reference object, which may have a complex 3D shape, for example, an anatomical part of a human body, while preserving the electromagnetic response from this reference object. In particular, the phantom device of the present invention may have a shape more practical and convenient to manufacture, while reproducing an electromagnetic response from the surface of the reference object.

Another aim of the present invention is to provide a phantom device which can reproduce the electromagnetic response from a surface of a reference object, such as a biological tissue, for example a human tissue, at least at two different predetermined frequencies, a first frequency within a first sub-frequency range, and a second frequency in a second sub-frequency range, said two sub-ranges in a frequency range from 6 GHz to at least 300GHz. As a non-limiting example, these two frequency sub-ranges can be 26-28GHz and 57-66GHz.

Another aim of the present invention is to provide a phantom device which is easier and less expensive to manufacture.

**Summary**

This disclosure improves the situation.

It is proposed a phantom device for reproducing at least one electromagnetic (EM) characteristic of a reference object made of an electromagnetic lossy medium, in particular a biological tissue, when illuminated by an EM wave with a predetermined frequency f1 emitted by an EM source, said phantom device comprising at least one unit structure, said unit structure comprising:

- at least one first dielectric layer comprising an upper surface face to the electromagnetic source and a bottom surface opposite to the upper surface,
- said upper surface being at least partly transparent to the electromagnetic waves emitted by the source;
- said bottom surface being at least partly reflecting for the electromagnetic waves transmitted through the first dielectric layer;
- said at least one first dielectric layer characterized by a complex dielectric permittivity of its bulk material $\varepsilon_1^* = \varepsilon_1' - j\varepsilon_1''$ having an absolute value in a range between 3 and 40;
- said at least one first dielectric layer further characterized by a thickness $T_1$ at least equal to 1/4 of the wavelength of the EM wave in the medium of the first dielectric layer.

In an embodiment, the reference object representing a human tissue, the complex dielectric permittivity $\varepsilon_1^*$ and the thickness $T_1$ of the first dielectric layer are jointly selected so as to reproduce at least the absolute value of the complex reflection coefficient from the surface of the reference object at least for the case of the normal incidence of the EM wave, said absolute value being in the range of 0.40 - 0.75 at the frequency f1 in the frequency range of 6GHz to 300GHz.

In a preferred embodiment, the at least one first dielectric layer further has the thickness $T_1$ smaller or equal to the penetration depth of the EM wave into the medium of said first dielectric layer and is at least partly transparent to the EM wave at frequency f1 emitted by the EM source.

In another embodiment, the phantom device further comprises at least one second layer, the first dielectric layer being positioned on the at least one second layer, said at least one second layer being made of a dielectric material, said first dielectric layer and said second dielectric layer jointly configured to reproduce at least the absolute value of the reflection coefficient from the surface of the reference object, with a relative contribution of a portion of the EM wave reflected from the bottom surface, at the interface between the first layer and the second layer, into the total reflection coefficient from the upper surface of the phantom device constituting at least 5%.

In a preferred embodiment, the second layer has a thickness $T_2$ selected such that the penetration depth of the electromagnetic wave into the medium of the second layer is at least equal to the thickness of said second layer, said first dielectric layer and said second dielectric layer being jointly configured to reproduce the absolute value of the complex reflection coefficient from the surface of the reference object, while remaining at least partly transparent to the incident EM wave.

In one further embodiment, the phantom device further comprises at least one second layer, the first dielectric layer being positioned on the at least one second layer, said at least one second layer being made of a composite medium, comprising a first fraction made of a conductive material having an electric conductivity $\sigma_2$ equal at least $10^2$ s/m and a second fraction made of a dielectric material, the volume ratio between the first fraction and the second fraction being selected in a range between 10% and 90% to provide a relative contribution of a portion of the EM wave reflected from the bottom surface, at the interface between the first layer and the second layer, into the total reflection coefficient from the upper surface of the phantom device constituting at least 5%.

In yet another embodiment, the at least one second layer is made of a conductive material, characterized by the electrical conductivity at least equal to $10^2$ S/m, with a thickness $T_2$ being larger than the penetration depth of the EM wave into said conductive material, said second layer further comprising at least one through hole forming a transparent zone for the EM wave, said at least one through hole having a surface area in the range of 10 to 90% of the total surface of the unit structure and being filled in with a dielectric medium, said at least one through hole having an arbitrary shape in xy-plane aligned with the bottom surface, defined by a contour line with the length at least equal to a half of the wavelength of the EM wave in the dielectric medium filling the at least one through hole or in the medium of the first layer.

In an embodiment, the first layer and the second layer are jointly configured to reproduce the at least two different values of the at least one EM characteristic of a reference object when illuminated by two different EM waves, a first EM wave with a frequency f1 within a first frequency subrange and a second EM wave with a frequency f2 within a second frequency sub-range, said two sub-ranges within the frequency range of 6GHz to 300GHz.

In one example, the thickness of the first dielectric layer is smaller than the penetration depth of the first EM wave with the frequency f1, and simultaneously being at least equal to the penetration depth of the second EM wave with the frequency f2.

In another embodiment, the phantom device further comprises a frequency selective layer being reflecting for a first incident electromagnetic wave at a first frequency f1 and being transparent to a second incident electromagnetic wave at a second frequency f2, the frequency selective layer being embedded in the at least one first layer or attached to the bottom surface of the at least one first layer.

In an embodiment, the phantom device comprises a plurality of unit structures for reproducing locally variable electromagnetic response of the reference object, each unit structure being configured to reproduce the at least one electromagnetic characteristic from a portion of the surface of the reference object.

In one example, the size, the shape and the composition of the layers forming each unit structure, and/or the distance between two adjacent unit structures are selected to obtain a continuous variation of the electromagnetic response along a surface of the phantom system.

In another example, the size, the shape and the composition of the layers forming each unit structure, and/or the distance between two adjacent unit cells are selected to obtain a discrete variation of the electromagnetic response along a surface of the phantom system.

In yet another example, each unit structure comprises a microelectromechanical switch configured to change the total thickness of the unit structure and/or the curvature of the top surface of the unit structure, or the effective complex permittivity of the first layer or of the second layer.

In another aspect, it is proposed a dosimetry system for measuring an electromagnetic dosimetry quantity related to an electromagnetic field emitted by an electromagnetic source, the dosimetry system comprising:

- a phantom device as defined above, comprising an upper surface face to the electromagnetic source and a bottom surface, said phantom device being at least partly transparent to the EM wave emitted by the electromagnetic source;
- at least one sensor attached to or arranged beneath the bottom surface and configured to measure a physical quantity related to the electromagnetic wave transmitted through the phantom device;
- a signal analyzing unit configured to analyze the signal transmitted from the at least one sensor, a processing unit configured to calculate the electromagnetic dosimetry quantities from the signal and a memory unit.

In one embodiment, the at least one sensor comprises an electromagnetic sensor operating at a frequency of the electromagnetic source.

In another embodiment, the at least one sensor comprises an electromagnetic sensor operating at a frequency different from that of the electromagnetic source, the device further comprising frequency converter element.

In another further embodiment, the at least one sensor comprises a thermal sensor.

**Brief Description of Drawings**

Other features, details and advantages will be shown in the following detailed description and on the figures, on which:

**Fig. 1**

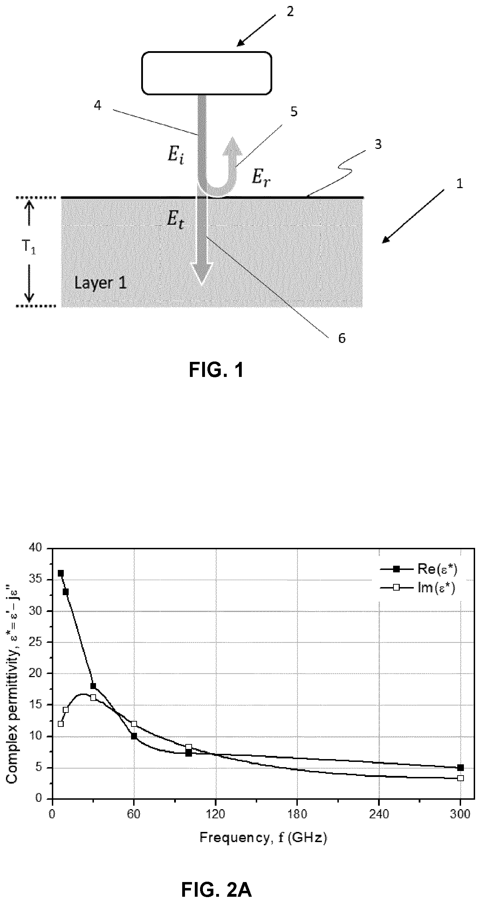

[Fig. 1] Figure 1 is a schematic illustration of the electromagnetic reflection from a layer made of a lossy material with a thickness larger than the penetration depth of the incident electromagnetic wave emitted by an electromagnetic source.

**Fig. 2A**
[Fig. 2A] Figure 2A represents the real and imaginary part of the complex permittivity of the human skin tissue versus frequency.

**Fig. 2B**

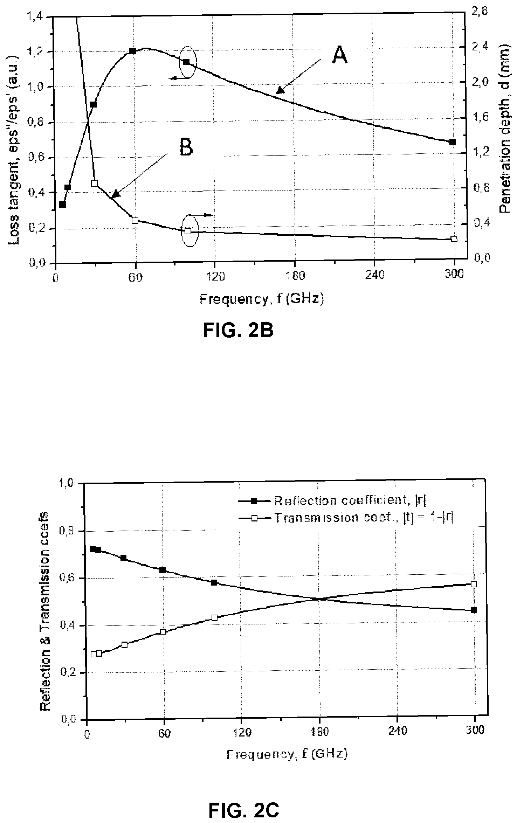

[Fig. 2B] Figure 2B represents the loss tangent in the medium of the skin tissue and the penetration depth of the electromagnetic wave at normal incidence on the human skin tissue versus frequency.

**Fig. 2C**
[Fig. 2C] Figure 2C represents the reflection and transmission coefficients at the interface of the human skin tissue versus frequency.

**Fig. 3**

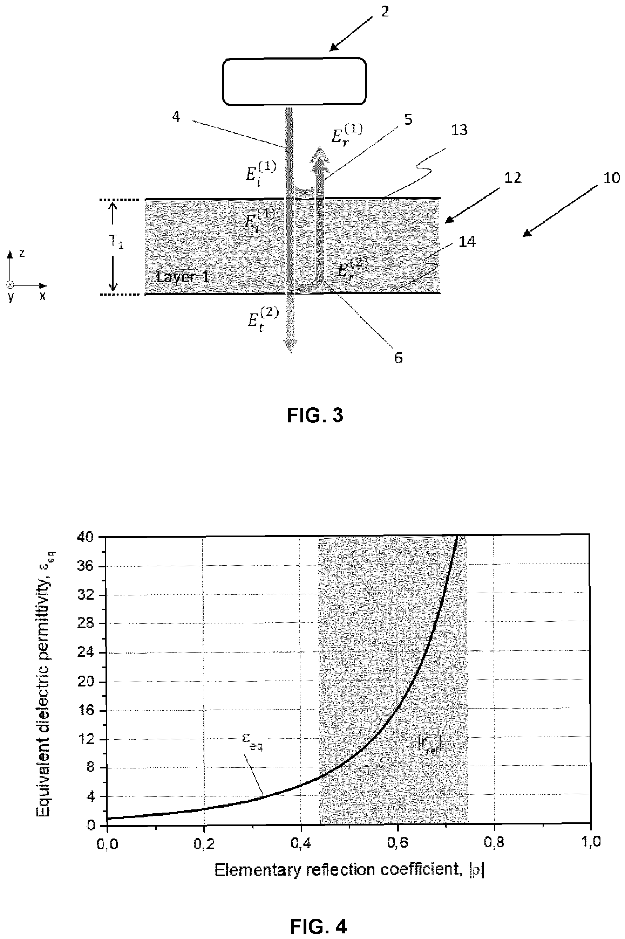

[Fig. 3] Figure 3 is a schematic illustration of a cross-sectional view of an embodiment of a phantom device comprising a single dielectric layer.

**Fig. 4**
[Fig. 4] Figure 4 represents the calculated value of the equivalent dielectric permittivity of a semi-infinite lossless dielectric medium versus the elementary reflection coefficient from its surface.

**Fig. 5A**

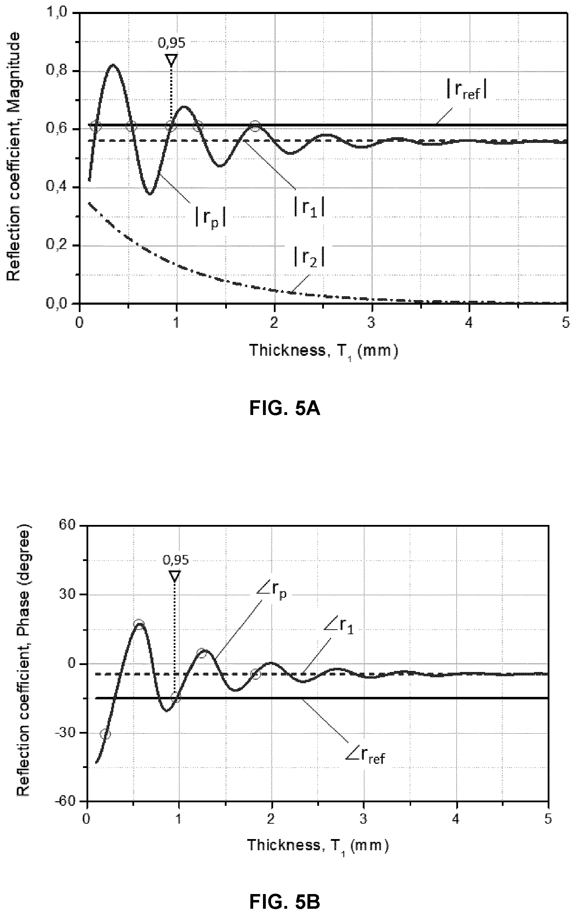

[Fig. 5A] Figure 5A represents the calculated absolute value of the complex reflection coefficient versus thickness of the first dielectric layer having a complex permittivity $\varepsilon_1^* = 11.68 - j2.92$ for the phantom device of figure 3.

**Fig. 5B**
[Fig. 5B] Figure 5B represents the calculated phase of the complex reflection coefficient versus thickness of the first dielectric layer for the same phantom device as in figure 5A.

**Fig. 5C**

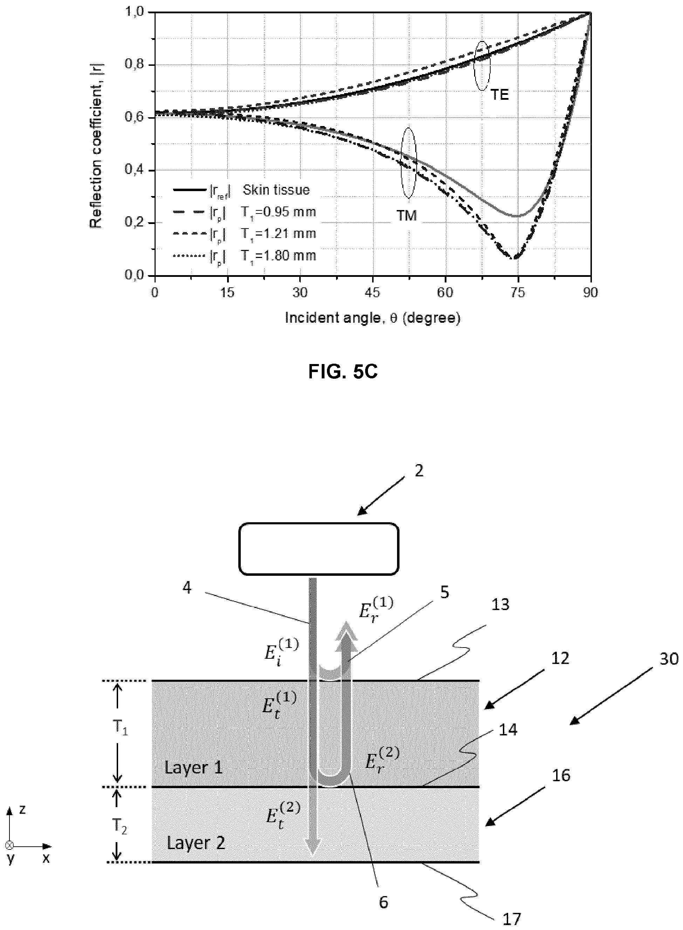

[Fig. 5C] Figure 5C represents the calculated absolute value of the complex reflection coefficient from the surface of the phantom device in figure 5A and 5B versus incident angle of the EM wave for different thickness values $T_1$ =0.95 mm, $T_1$ =1.21 mm and $T_1$ =1.80 mm.

**Fig. 6**

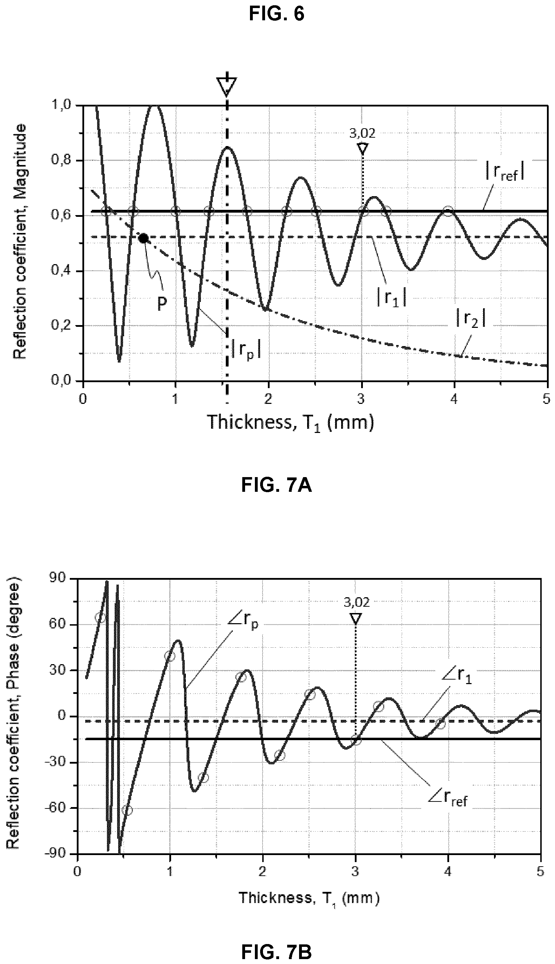

[Fig. 6] Figure 6 is a schematic illustration of a cross-sectional view of another embodiment of a phantom device for electromagnetic dosimetry comprising a first dielectric layer and a second layer.

**Fig. 7A**
[Fig. 7A] Figure 7A represents the calculated absolute value of the reflection coefficient from an example of phantom device of figure 6 having a dielectric layer complex permittivity $\varepsilon_1^* = 10.03 - j1.3$ versus thickness of the first dielectric layer.

**Fig. 7B**
[Fig. 7B] Figure 7B represents the phase of the complex reflection coefficient versus thickness of the first dielectric layer for the phantom device as in figure 6.

**Fig. 7C**

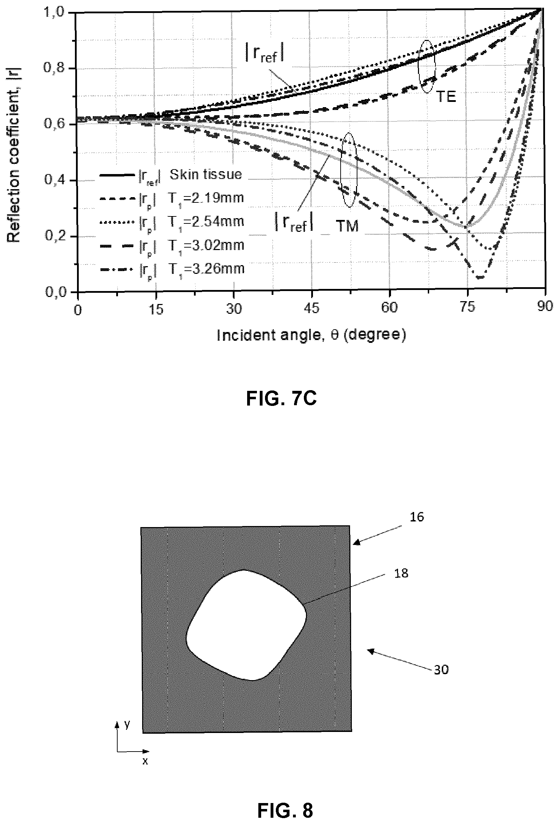

[Fig. 7C] Figure 7C represents the absolute value of the complex reflection coefficient from the surface of the phantom device in figure 7A and 7B versus incident angle of the EM wave for different thickness values $T_1$ =2.19 mm, $T_1$ =2.54 mm, $T_1$ =3.02 mm and $T_1$ =3.26 mm.

Fig. 8
**[Fig. 8]** Figure 8 is a schematic illustration of a cross-sectional view of a second layer made of composite medium according to an embodiment comprising a through hole.

**Fig. 9A**

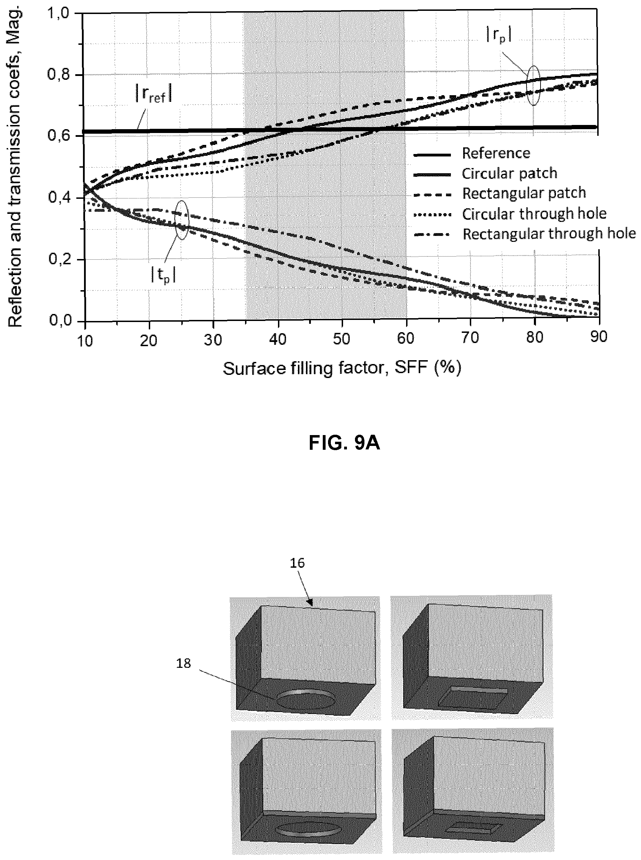

[Fig. 9A] Figure 9A represents transmission and reflection coefficients of the phantom device of figure 6 comprising a first layer having a complex permittivity $\varepsilon_1^* = \quad 10.03 - j1.3$ and a second layer made of a composite material with inclusions of a conductive material ($\sigma_2 = 10^7$, $T_2 = 0.1$mm) having a rectangular (solid line) or circular (dashed line) shape versus the surface filling factors of the inclusions (SFF).

**Fig. 9B**

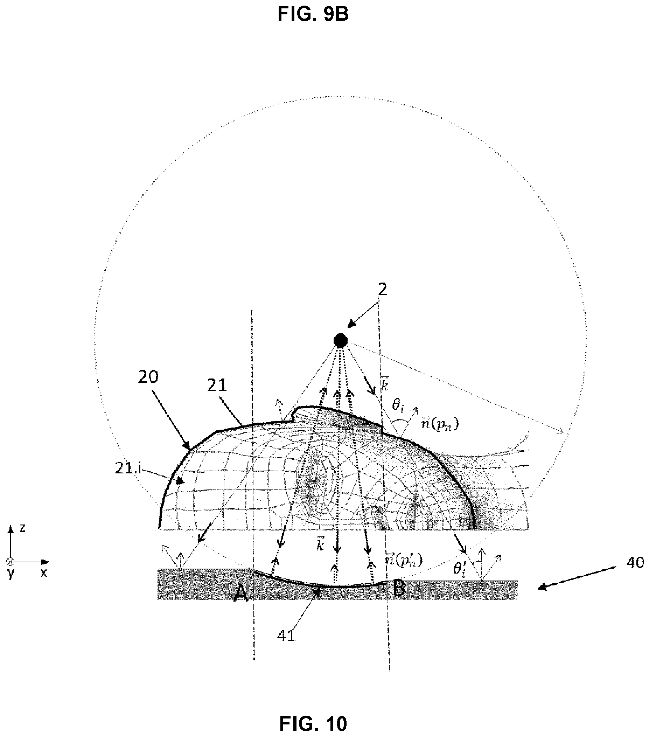

[Fig. 9B] Figure 9B represents four different configurations of the unit structures of the device in figure 9A, characterized in different form of the inclusions referred as : a circular patch (1), a rectangular patch (2), a circular through hole (3) and a rectangular through hole (4).

**Fig. 10**
[Fig. 10] Figure 10 is a schematic illustration of a cross-sectional view of yet another embodiment of a phantom device for electromagnetic dosimetry reproducing an example of reference object in a form of a human head exposed to electromagnetic waves from a source.

**Fig. 11A**

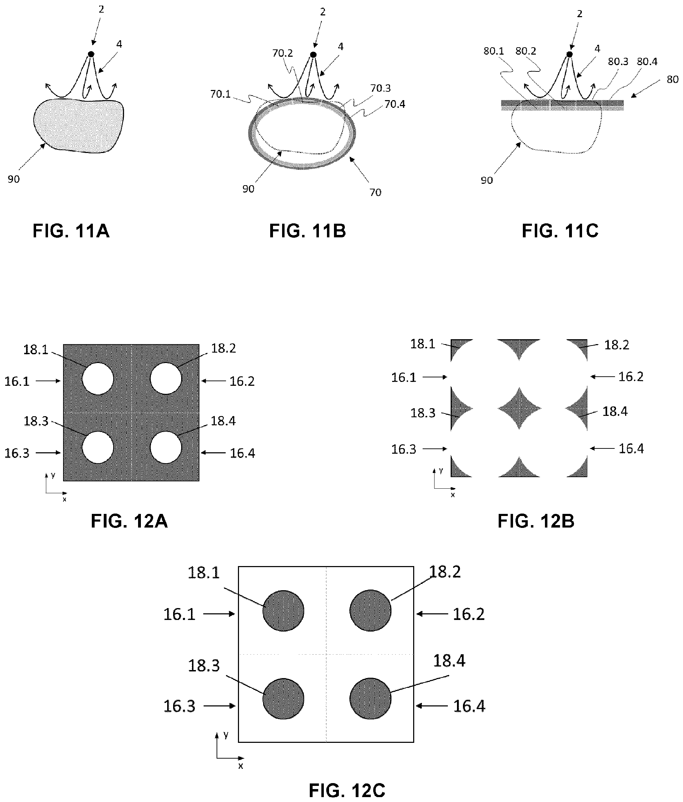

[Fig. 11A] Figure 11A represents a schematic illustration of a 3D reference object having a complex geometric shape.

**Fig. 11B**
[Fig. 11B] Figure 11B represents a schematic illustration of a phantom device comprising a plurality of unit structures forming a 3D primitive shape while reproducing the electromagnetic response of the 3D reference object of figure 11A.

**Fig. 11C**
[Fig. 11C] Figure 11C represents a schematic illustration of a phantom device comprising a plurality of unit structures forming a 2D planar structure while reproducing the electromagnetic response of the 3D reference object of figure 11A.

**Fig. 12A**
[Fig. 12A] Figure 12A represents a schematic illustration of a cross-sectional view of a phantom device comprising four unit structures in a horizontal XY-plane at Z crossing a second layer made of a composite medium, each second layer comprising a circular through hole according to an embodiment.

**Fig. 12B**
[Fig. 12B] Figure 12B represents a schematic illustration of a cross-sectional view of a phantom device comprising four unit structures in a horizontal XY-plane at Z crossing a second layer made of a composite medium comprising a circular through hole with the surface ratio of about 80% of the surface of the unit structure so that the through holes of the adjacent unit structure merge.

**Fig. 12C**
[Fig. 12C] Figure 12C represents a schematic illustration of a cross-sectional view of a phantom device comprising four unit structures in a horizontal XY-plane at Z crossing a second layer made of a composite medium, each unit structure comprising a conductive circular inclusion in a conductive medium according to an embodiment.

**Fig. 13A**

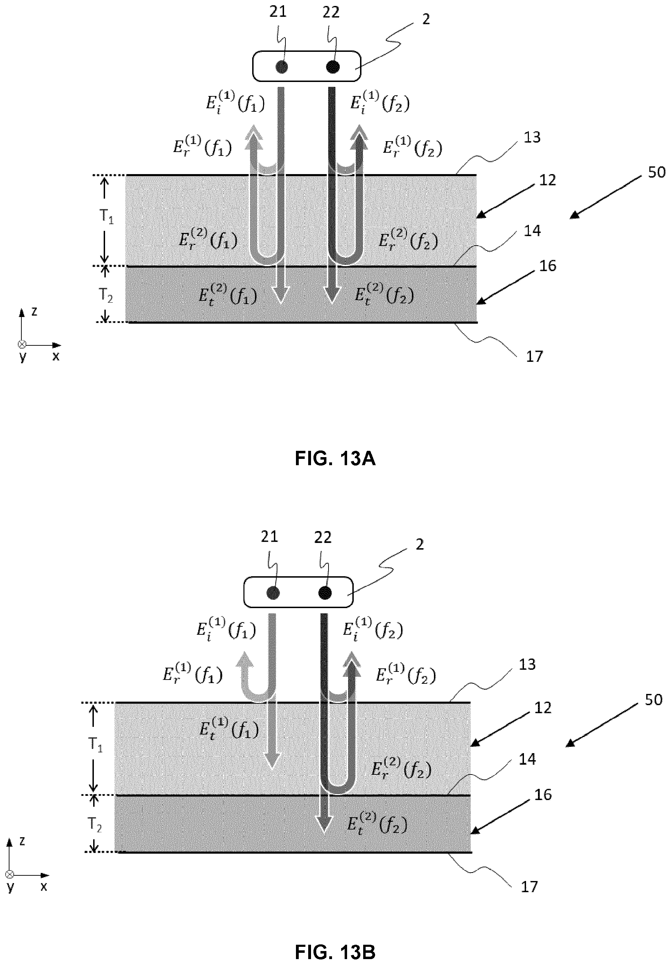

[Fig. 13A] Figure 13A is a schematic illustration of a cross-sectional view of an embodiment of a phantom device for electromagnetic dosimetry configured to reproduce two different electromagnetic responses at two frequency sub-bands.

**Fig. 13B**
[Fig. 13B] Figure 13B is a schematic illustration of a cross-sectional view of another embodiment of a phantom device for electromagnetic dosimetry configured to reproduce two different electromagnetic responses at two frequency sub-bands.

**Fig. 14**

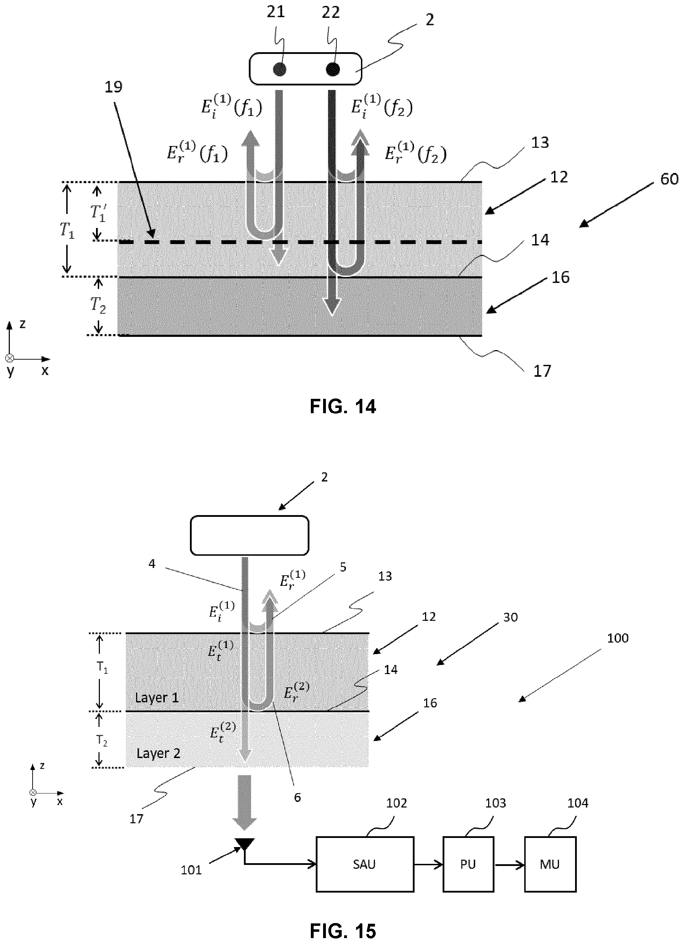

[Fig. 14] Figure 14 is a schematic illustration of a cross-sectional view of yet another embodiment of a phantom device for electromagnetic dosimetry comprising a frequency selective layer embedded in the first dielectric layer to reproduce two electromagnetic responses at two frequencies.

**Fig. 15**

[Fig. 15] Figure 15 a schematic illustration of a cross-sectional view of an embodiment of a system for electromagnetic dosimetry comprising a phantom device of figure 6.

**Description of Embodiments**

The terms "top," "bottom," "over," "under," "between," "on", and other similar terms as used herein refer to a relative position. The relative position of the terms does not define or limit the layers to a vector space orientation.

To facilitate understanding, identical reference numerals have been used, where possible, to designate identical elements that are common to the figures. It is contemplated that elements disclosed in one embodiment may be beneficially utilized on other embodiments without specific recitation.

In the present disclosure, the term "layer" refers to a structure having a constant thickness or a variable thickness. The layer may be planar or curved. In particular, the layer may be shaped to reproduce a 3D form of a part of a human body, e.g., head or hand. The layer may be shaped to form a closed loop such a side wall of a cylindrical object.

In the present disclosure, the term "dosimetric quantity" refers to any metric used to quantify the electromagnetic exposure levels, including power density, energy density, specific absorption rate, specific energy absorption, electric field strength, magnetic field strength, electric voltage or current defined with respect to an averaging surface area or volume.

In the present disclosure, the term "effective permittivity" can be understood in the sense of a weighted average of the dielectric permittivity of different fractions of a compound material, rather than that of a bulk material representing a uniform layer. For a compound material with inclusions having size smaller than the wavelength, it can be approximately defined as a volume ration of the two fractions. In case of a larger size inclusions, the effective value can be understood as the impedance matching condition (defined by the ratio of the complex permittivity of the two adjacent media). For instance, it can be done by varying the size of the through holes or by varying size of inclusions embedded in the medium.

In the present disclosure, the term "reference object" refers to a structure characterized by a predetermined electromagnetic response when illuminated by an electromagnetic wave, emitted by an electromagnetic source, characterized by a predetermined frequency, polarization and incident angle defined with respect to the surface normal vector. Hereafter, the electromagnetic response can refer to any metric used to quantify the electromagnetic field scattered by said reference object, including the absolute value (i.e. magnitude) of the complex reflection coefficient, or the phase of the complex reflection coefficient, or the scattering cross-section, or the scattering pattern that can be defined at least for a portion of the surface area of the reference object and/or a portion of the electromagnetic field incident on said surface area. Said portion of the electromagnetic field may represent one polarization, or a given angular range of the incident angle, or a frequency sub-range in a wider frequency range. Said predetermined electromagnetic response of a reference object may be determined, for example, by electromagnetic measurements fulfilled under the realistic use case illumination conditions or from the literature. A non-limiting list of examples of the reference objects considered by this invention may include any biological tissues of a living creature (e.g. human, birds, animal), any anatomical part of the body of said living creature (e.g. head, torso, arm, hand, leg, etc.), or any other reference object that can be characterized by its shape and a known or target electromagnetic response when illuminated by an electromagnetic wave with a predetermined frequency, polarization and incident angle. In a preferred embodiment, the reference object represents a biological tissue, such as human skin tissue, whose electromagnetic response in known from the literature.

With reference to figure 1, the electromagnetic response from the surface of a reference object, for example a human skin, is illustrated. The human skin is represented by a structure comprising a layer of a lossy material with a thickness $T_1$ and extending along a plane (XY). The structure is surrounded by a host medium which can be the air.

An electromagnetic field source 2 is disposed at a distance from the upper surface of the reference object. The source 2 may be a mobile phone or any other wireless device including wearable or portable. The source comprises one or more antennas or radiating structures connected to the body of the source for transmitting and receiving electromagnetic signal.

As shown in figure 1, an electromagnetic wave 4 at a predetermined frequency, provided by the source 2, illuminates the upper surface of the human skin. A portion of the incident electromagnetic wave 5 is reflected by the upper surface 3 of the layer 1 and a portion of the incident electromagnetic wave 6 penetrates the layer 1 and is absorbed in the lossy medium of the layer 1.

The figures 2A to 2C illustrate the electromagnetic properties of a human skin tissue versus frequency of the incident electromagnetic wave.

The figure 2A illustrates real and imaginary parts of the complex permittivity versus frequencies from 6 GHz to 300 GHz.

The curve A of the figure 2B illustrates the growth of the loss tangent in the frequency range of 6 to 60 GHz defined as the ratio between the imaginary and real parts of the complex permittivity represented in figure 2A. The curve B shows that the penetration depth decreases versus frequency. The penetration depth is less than 0.5mm for frequencies above 60 GHz.

The figure 2C illustrates reflection and transmission coefficients at the skin surface versus frequency of the electromagnetic wave at normal incidence on the upper surface 3 of the layer 1, at the interface between the air and the layer.

The present invention proposes a new phantom device which reproduces the electromagnetic response of a reference object made of an electromagnetic lossy medium, such as a biological tissue, when its surface is illuminated by an electromagnetic wave emitted by an electromagnetic source 2.

With reference to figure 3, a phantom device 10 according to an embodiment of the present disclosure will now be described.

The phantom device 10 comprises a dielectric layer 12 extending in a horizontal plane (XY). In the embodiment of figure 3, the phantom device 10 comprises a dielectric layer 12, which defines two main surfaces, a top surface 13 facing the electromagnetic source 2 and a bottom surface 14 opposite with respect to the electromagnetic source 2.

In another embodiment, the phantom device may comprise more than one dielectric layer.

The upper surface 13 is at least partly transparent to the incident electromagnetic wave 4. The bottom surface 14 is at least partly reflecting for the electromagnetic wave 4 emitted by the source and transmitted through the layer 12.

With reference to figure 3, the electromagnetic wave 4 emitted by the source 2 is incident on the upper surface 13 of the first dielectric layer 12 and the incident electromagnetic wave is partially reflected by the upper surface 13. The reflected EM wave is designated by the numeric reference 5. A portion of the EM wave that is not reflected by the upper surface 12, at the air-dielectric interface, propagates through the dielectric layer 12, from the upper surface 13 to the bottom surface 14, and is partly absorbed in the dielectric medium of the layer 12. The electromagnetic wave transmitted through the first dielectric layer 12 is then at least partially reflected by the bottom surface 14. The reflected EM wave is designated by the numeric reference 6.

The phantom device 10 can reproduce a desired electromagnetic response from the upper surface of a reference object by using the interplay between the wave 5 reflected from the upper surface 13 and the wave 6 reflected from the bottom surface 14. This may be achieved via a constructive or destructive interference between the two waves leading to the more or less pronounced reflection from the upper surface 13 of the phantom device. The complex reflection coefficient from the upper surface 13 is defined as follows:

$$r_p = \frac{E_r^{(1)}}{E_i^{(1)}} = \frac{E_r^{(s_1)} + E_r^{(s_2)}}{E_i^{(1)}} = r_1 + r_2 = \rho_1 + \rho_2 * (1 - |\rho_1|^2) * e^{-2\gamma_1 T_1}, \tag{1}$$

where $E_r^{(s_1)}$ and $E_r^{(s_2)}$ are the two reflected EM waves associated with the upper surface 13 and the bottom surface 14, $\rho_1$ and $\rho_2$ are respectively the elementary reflection coefficients associated with the top and bottom surfaces defined as a function of the EM field polarization and incident angle, $T_1$ is the thickness of the layer 12, and $\gamma_1 = \alpha_1 + j\beta_1$ is the propagation constant describing the attenuation $\alpha_1$ and the phase $\beta_1$ factors in the medium of the layer 12 which are defined as follows:

$$\alpha_1 = 2\pi f \left\{ \frac{\mu_1 \varepsilon_1'}{2} \left( \left[ 1 + \left( \frac{\varepsilon_1''}{\varepsilon_1'} \right)^2 \right]^{1/2} - 1 \right) \right\}^{1/2} \tag{2}$$

$$\beta_1 = 2\pi f \left\{ \frac{\mu_1 \varepsilon_1'}{2} \left( \left[ 1 + \left( \frac{\varepsilon_1''}{\varepsilon_1'} \right)^2 \right]^{1/2} + 1 \right) \right\}^{1/2} \tag{3}$$

According to the relation (1), the first reflected wave $E_r^{(s_1)}$ is determined by the elementary reflection coefficient $\rho_1$ from the top surface 13 at the interface between the dielectric layer 12 and the host medium above the layer 12. The second reflected wave $E_r^{(s_2)}$ is associated with the elementary reflection coefficient $\rho_2$ from the bottom surface 14 at the interface between the dielectric layer 12 and the host medium below the layer 12. The second reflected wave $E_r^{(s_2)}$ is determined by the complex permittivity of the layer 12, the thickness $T_1$ of the layer 12, and the complex permittivity of the host medium below the layer 12. Thus, it is possible to adapt the amplitude and phase of the second reflected wave by varying the thickness $T_1$ and/or the complex permittivity $\varepsilon_1^*$ of the layer 12, and/or the complex permittivity of the host medium below the layer 12.

The phantom device of the present disclosure is defined by a specific combination of the complex dielectric permittivity $\varepsilon_1^*$ and thickness $T_1$ to generate a desired electromagnetic response, such as the complex reflection coefficient $r_p$, or only the magnitude of the complex reflection coefficient $|r_p|$, from the surface of the layer 12 as required to reproduce the corresponding electromagnetic response from the upper surface of a reference object, for example a human tissue.

In a preferred embodiment, the dielectric layer 12 is characterized by a complex dielectric permittivity $\varepsilon_1^* = \varepsilon_1' - j\varepsilon_1''$ having an absolute value in a range between 3 and 40, that may correspond to a high index ( $\varepsilon_1'$ in the range between 7 and 40) and relatively low-loss (*tanδ* < 0.05) material from a group of materials comprising semiconductors (such as crystalline silicon), oxides (such as metal oxides and ferroelectric oxides), ceramics (such as alumina and doped perovskite materials) and compound ceramic/polymer materials, or, on the opposite, to a material with a moderate value of the real part of the complex permittivity ( $\varepsilon_1'$ in the range between 3 and 16) and relatively high losses (*tanδ* < 0.5) from a group materials comprising various types of glass, plastic (such as teflon, kapton, polymethylmethacrylate, polypropylene, polyamide, polycarbonate), resin (such as epoxy) or organic polymer materials (such as PDMS) doped with electrically conductive additives (such as carbon, graphene, silver and alike in the form of microparticles, nanorods, nanowires, etc.) to achieve a required value of the effective complex dielectric permittivity. More specifically, the real part of the effective complex permittivity of a compound dielectric medium of the first dielectric layer 12 may be selected in the range between 4 and 12 with a loss tangent in the range between 0.01 and 0.3.

In this preferred embodiment, the dielectric layer 12 is further characterized by a thickness $T_1$ in a range between 1/10 and 10 wavelengths, preferably in a range between 1/4 and 4 wavelengths, of the electromagnetic wave with the frequency f1 in the medium of the layer 12.

Advantageously, it is possible to select the effective complex dielectric permittivity $\varepsilon_1^*$ and thickness $T_1$ of the layer 12 to reproduce the absolute value (i.e. magnitude) of the complex reflection coefficient from the reference object to be simulated with a tolerance defined by a threshold value $T_r$:

$$\left(|r_p| - |r_{ref}|\right)^2 / |r_{ref}|^2 \leq T_r, \qquad (4)$$

Where $r_{ref}$ represents the complex reflection coefficient from the upper surface of the reference object, in other words the target value to be achieved. $r_p$ represents the complex reflection coefficient from the upper surface of the phantom device in figure 3, as defined by equation (1), and $T_r$ represents the relative deviation acceptable from the target value and the value reproduced by the phantom device. In a preferred embodiment, $T_r$ is between $10^{-4}$ and $10^{-2}$.

The dielectric layer 12 may be made of dielectric material beneficially dopped with conductive fillers, such as particles or inserts or a material comprising inclusions of another material characterized by a different value of the complex permittivity. The material of the layer, the type of the doping and its volume ration is chosen to have the complex dielectric permittivity $\varepsilon_1^*$ required and combined with the thickness $T_1$ of the layer 12 to reproduce the desired electromagnetic response, from the top surface 13 of the layer 12, such as the complex reflection coefficient or its magnitude.

In one embodiment, the reference object is a human skin tissue, the complex dielectric permittivity $\varepsilon_1^*$ and the thickness $T_1$ of the first dielectric layer 12 are selected so as to reproduce the absolute value of the complex reflection coefficient from the surface of the human tissue. For example, the absolute value of the complex reflection coefficient is in the range of 0.40 - 0.75 at a frequency f1 in the frequency range of 6GHz to 300GHz.

The phantom device can be used to create a unit structure reproducing the electromagnetic reflection coefficient of a biological tissue. Advantageously, the phantom device can be used to create a unit structure reproducing the electromagnetic response of any other planar or 3D objects characterized by a relatively high reflectivity (in the range of $|r_{ref}| \geq 0.4$) when exposed to non-ionizing electromagnetic radiation with a predetermined frequency in the frequency range of 1GHz to 10THz, such as 6GHz to 300GHz. For example, the phantom device of the present disclosure may be beneficially used to reproduce the electromagnetic response from the surface of a reference object, e.g. vehicles, drones, robots, or elements of the indoor environment having at least one reflecting surface, exposed to non-ionizing EM radiation. In other words, the phantom device of the present disclosure can be used to produce a tailored EM response from a surface, both in the reflection and transmission modes, according to a given reference pattern that can be measured or predicted analytically by solving a corresponding EM scattering problem.

In one embodiment, the phantom device comprises one unit structure which may form a 2D planar structure as illustrated in figures 3 and 6. In another embodiment, and with reference to figure 10 and figure 11B, the phantom device may comprise a plurality of unit structures which form a complex shape reproducing the form of a reference object.

The figure 4 represents the equivalent dielectric permittivity of a semi-infinite lossless dielectric medium versus the elementary reflection coefficient p from the air/dielectric interface at the upper surface calculated as

$$\varepsilon_{eq} = \left(\frac{1+|\rho|}{1-|\rho|}\right)^2 . \tag{5}$$

The shadow zone represents the range of values of the elementary reflection coefficient from the reference object, such as biological tissue, represented in figure 2C. For common low-loss dielectric materials with equivalent dielectric permittivity below 12 (such as Teflon, rexolite, quarts, silicon), the reflection from the upper surface, represented by the elementary reflection coefficient, does not exceed 0.55, which is not sufficient to cover the full range of desired values of the elementary reflection coefficient from the surface of the reference object, such as biological tissue. Moreover, the limited choice of common materials allows only for a discrete set of feasible values of the elementary reflection coefficient. By replacing the semi-infinite medium with a dielectric layer 12, providing a second reflecting surface, such as the bottom surface 14, the magnitude of the reflection coefficient from the upper surface 13 can be nearly doubled for the thickness of the layer being multiple of a half wavelength of the incident electromagnetic field in the medium of the layer. On the opposite, for a quarter-wavelength thickness, the magnitude of the complex reflection coefficient can be reduced to a very small level, subject of the propagation loss in the layer medium experienced by the portion of the wave reflected by the bottom surface 14. By varying the thickness and the effective complex permittivity of the layer medium, one skilled in the art can find a combination of these parameters resulting in the required value of the reflection coefficient, equal to that of a reference object, such a biological tissue.

The figure 5A represents the magnitude of the complex reflection coefficient from an exemplary phantom device versus the thickness of the layer 12. The layer 12 is made of compound characterized by an effective complex dielectric permittivity $\varepsilon_1^* = 11.68 - j2.92$. As predicted by the data represented in figure 5A, there is a plurality of thickness values highlighted by circular marks that allow the phantom device to reproduce the magnitude of the complex reflection coefficient from the human skin tissue at 60 GHz ($|r_{ref}|$= 0.615) illustrated by the solid horizontal line. These values appear at the intersection of the oscillating solid curve, representing the magnitude of the phantom reflection coefficient ($|r_p| = |r_1 + r_2|$), defined by the sum of the two terms describing the partial contributions of the two waves, a first one reflected from the upper surface (13) and a second one reflected from the bottom surface (14), defined by equation 1.

The figure 5B represents the phase of the complex reflection coefficient versus the thickness of the layer 12 for the same exemplary phantom device of figure 5A, superimposed with that of the reference object, illustrated by the solid horizontal line. The circular marks indicate the set of thickness values that, according to figure 5A, provide the same magnitude of the reflection coefficients. One may see that this set of thickness values includes one value ($T_1$=0.95) that simultaneously provides the desired values of the phase and magnitude of the complex reflection coefficient, as that of the reference object, such as the biological tissue when illuminated by an electromagnetic wave with frequency 60GHz. At the same time, the other values in the set of thickness values highlighted by circular marks, allows one to reproduce the magnitude of the reflection coefficient, while providing different phase response. As explained in the following, this functionality can be beneficially used as a mean to control angular scattering characteristics of the phantom device reproducing the magnitude of the reflection coefficient of a reference object, such as an anatomical part of the human body. Thus, in a configuration wherein the phantom device comprises a plurality of unit structures as illustrated below in figure 11B and figure 11C, reproducing the same magnitude of the reflection coefficient, it is possible to obtain different phase responses by selecting the appropriate set of thickness values for the layer 12. This allows to vary the angle of reflection from the surface of a planar phantom device to reproduce the scattering characteristics of a 3D shaped reference object.

The figure 5C represents the magnitude of the complex reflection coefficient versus the incident angle of the electromagnetic wave with TE and TM polarizations for three different thickness values from the set of thickness values indicated by circular marks in figure 5A. As predicted by the data represented in figure 5C, the phantom device in all three configurations provides the angular response similar to that of the reference object. In all three cases, the perfect match is provided at least for one polarization and for at least a certain angular range. In particular, in case of the phantom device with the thickness Ti=0.95, the accuracy threshold of $T_r = 10^{-4}$ is provided at least for TM polarization in the angular range of 0 to 45 degree, whereas for the two other configurations, the same threshold is provided in the entire angular range for TE polarization and at least in the range of 0 to 30 degree for the case of TM polarization.

Thus, the phantom device of the present disclosure may be specifically designed to reproduce the electromagnetic response of a reference object made of an electromagnetic lossy medium, such as a biological tissue, when its surface is illuminated by an electromagnetic wave with predetermined frequency emitted by an electromagnetic source while providing an electromagnetic transparency of the phantom structure to the electromagnetic wave incident on the surface of the phantom device in a range of 6 GHz to 300 GHz. In other words, the phantom device of the present disclosure can reproduce the complex reflection coefficient from the surface of the reference object, e.g., human tissue, while providing a partial transmission for the incident electromagnetic wave through the whole structure of the phantom device.

In the embodiment of figure 3, the layer 12, which forms the phantom structure, can reproduce the reflection response from the upper surface 13 of the layer 12, while providing a certain electromagnetic transparency (or a transmittance) for the incident electromagnetic wave emitted by the electromagnetic source at a frequency f1. Thus, a portion of the electromagnetic wave is transmitted through the layer 12. In one approximation, the transmission coefficient through the layer 12 may be estimated as a function of the elementary reflection coefficients at its upper surface 13 and bottom surface 14, and the propagation constant, defined by equation 2 and 3, as follows:

$$ t_p = (1 - \rho_1) * (1 - \rho_2) * e^{-\gamma_1 T_1} \qquad (6) $$

For the phantom device in figure 5A with the thickness $T_1$=0.95mm highlighted by a triangular mark, the equation 6 predicts the magnitude of the transmission coefficient through the phantom device of about 30%, i.e. $|t_p| \approx 0.3$.

In a preferred embodiment, the first dielectric layer 12 is characterized by a transmission coefficient having an absolute value (i.e. magnitude) being at least equal to 0.1. In other words, the amplitude of the electromagnetic wave transmitted through the dielectric layer shall be at least 10% of the electromagnetic wave incident on the top surface 13 of the phantom device.

In such embodiment, the phantom device can be used to create a 2D planar phantom structure reproducing the magnitude, or both magnitude and phase, of the complex reflection coefficient of the human tissue exposed to non-ionizing electromagnetic radiation emitted by an electromagnetic source. For example, the electromagnetic source may be a portable wireless device operating in the microwave range above 1GHz, and in particular, at frequencies above 6 GHz. Such a semi-transparent phantom device can be used in a dosimetry system for the exposure limit compliance testing of wireless devices. The proposed phantom device can substitute the human tissue which is characterized by high electromagnetic losses ($\tan \delta \geq 0.3$) to increase the penetration depth of the electromagnetic wave into the phantom device and/or the amount of power transmitted through the structure of the phantom device, improving thus the sensitivity and accuracy of the measurement, even at frequencies above 6GHz. An embodiment of dosimetry systems is described below with reference to figure 15, at least one sensor is positioned beneath the dielectric layer 12 and adapted to measure a physical quantity related to the electromagnetic wave transmitted through the dielectric layer 12.

The figure 6 is a schematic illustration of a cross-sectional view of another embodiment of a phantom device 30.

The device 30 comprises a first dielectric layer 12 and a second layer 16 characterized by a complex permittivity $\varepsilon_2^* = \varepsilon_2' - j\varepsilon_2'' = \varepsilon_2' - j\sigma_2/2\pi f$. The first dielectric layer 12 is on the second layer 16.

The electromagnetic wave 4 emitted by the source 2 is incident on the top surface 13 of the first dielectric layer 12 and the incident electromagnetic wave is partially reflected by the upper surface 13. The reflected EM wave is designated by the numeric reference 5. A portion of the EM wave that is not reflected by the upper surface 13, at the air-dielectric interface, propagates through the dielectric layer 12, from the upper surface 13 to the bottom surface 14. The electromagnetic wave transmitted through the first dielectric layer 12 is then partially reflected by the bottom surface 14. The reflected EM wave is designated by the numeric reference 6. A portion of the incident electromagnetic wave at the interface between the first layer 12 and the second layer 13 is transmitted through the second layer 16.

In one embodiment, this second layer 16 is made of a material characterized by the electrical conductivity $\sigma_2$ in a range between $10^2$ s/m and $10^7$ s/m. This second layer allows to adjust, increase or decrease the reflection from the bottom surface 14, depending on the ratio between the permittivity of the medium of the layer 12 and that of the layer 16. Thus, due to the presence of this second layer 16 made of a conductive material, a large portion of the incident wave is reflected by the bottom surface 14, and, if the attenuation of the electromagnetic wave in the first layer 12 is not high, the amplitude of the second wave may exceed that of the first wave $E_r^{(s_2)} > E_r^{(s_1)}$ and the contribution from the second wave, reflected from the bottom surface 14, will be dominant.

Advantageously, it is possible to adapt the elementary reflection coefficient $\rho_2$ by varying the thickness $T_2$.

In an embodiment with the thickness $T_2$ larger than the penetration depth of the electromagnetic wave in the medium of the layer 16, the reflection from the bottom layer 14 is solely defined by the complex dielectric permittivity of the layer 16 bulk material.

In another embodiment with the thickness $T_2$ smaller than the penetration depth of the electromagnetic wave in the medium of the layer 16, the reflection from the bottom surface 14 can be predicted as a function of the sheet resistance of the conductive layer that can be directly measured or taken from literature as a function of the bulk material conductivity and thickness of the layer 16.

In yet another embodiment, with the thickness $T_2$ comparable with the penetration depth of the electromagnetic wave in the medium of the layer 16, the reflection from the bottom surface 14 can be defined as a function of bulk material dielectric permittivity and thickness, similarly as for the first. Advantageously, the second layer can be sandwiched between a first dielectric layer and a third layer. Thus, for a selected material of the second layer 16, it is possible to vary the amplitude and phase of the second wave $E_r^{(s_2)}$ by adjusting the thickness of the second layer 16, and the complex permittivity and thickness of the third layer.

In an embodiment, the thickness of the first layer 12 is advantageously selected nearly equal to a threshold value $T_1 \sim T_1^{eq}$, where $T_1^{eq}$ is the equilibrium point at which the two waves reflected from the upper and from the bottom surfaces (as defined in equation 1) have the same amplitude (i.e. $\left| E_r^{(s_1)} \right| = \left| E_r^{(s_2)} \right|$). This thickness is denoted in figure 7A by the point P, whose value that can be estimated as follows:

$$T_1^{eq} = \frac{1}{2\alpha_1} \ln \left( \frac{\rho_1}{\rho_2 * (1 - |\rho_1|^2)} \right) \qquad (7)$$

In a preferred embodiment, the thickness of the first layer 12 can be beneficially selected to be slightly larger than the equilibrium point $(T_1 \gtrsim T_1^{eq})$ because it can provide a wider dynamic range of the phantom reflection coefficient variation from nearly zero to a two-fold value of the elementary reflection coefficient from the top surface of the layer 12, as well as a slower phase variation that leads to a wider performance bandwidth corresponding to the frequency range in which the magnitude of the reflection coefficient from the phantom device ($r_p$) is equal to that of the skin ($r_{ref}$) with a certain acceptable tolerance as defined in equation 4.

The figures 7A and 7B represent the magnitude and phase of the complex reflection coefficient form the surface of an exemplary phantom device of figure 6 versus the thickness of the first layer 12. The first layer 12 is made of a composite material characterized by $\varepsilon_1^* = 10.03 - j1.3$. The second layer 16 is made of a material with a high electric conductivity $\sigma_2 = 10^7$ S/m.

As shown in figure 7A, the behavior of the curve representing the magnitude of the complex reflection coefficient is similar to that observed in figure 5A for a single layer structure 12. The absolute value of the complex reflection coefficient oscillates and tends to the value of the elementary reflection coefficient of the first layer $r_1 = \rho_1$. The point P represents the equilibrium point as defined by equation (7).

Due to the stronger reflection from the second layer 16, the initial value of the reflection coefficient $|r_p|$ exceeds the target reference value of the human skin tissue at 60 GHz (rref) which is represented by the thick horizontal line. However, the target value of the reflection of the human tissue can still be provided in several points highlighted by circular marks, for example for $T_1$ = 0.36 mm or $T_1$ = 0.47 mm or 1.06 mm, etc.

The presence of the second conductive layer 16 contributes to the control of the elementary reflection coefficient at the bottom surface 14. In other words, the second conductive layer 16 provides control of the amplitude and phase of the second reflected wave. The maximum of the total reflection is obtained when the two waves $E_r^{(s_1)}$, $E_r^{(s_2)}$ are in phase. This condition can be used to determine the adapted value of the thickness of the first layer 12 and a suitable value for the complex permittivity of the first layer 12.

As predicted by figure 7B representing the phase of the complex reflection coefficient, the same target value of the magnitude of the reflection coefficient may correspond to different phase values in the range of ±60°. The corresponding thickness values are highlighted with circular marks same as in figure 7A. Note that, same as in case of the phantom device in figure 5, the set of the highlighted thickness values include one value ($T_1$ = 3.02mm) that provides the same magnitude and phase of the complex reflection coefficient as of the reference object, such as human skin tissue at 60 GHz.

The figure 7C represents the reflection coefficient from the surface of the same exemplary phantom device as in figures 7A and 7B versus the incident angle of the electromagnetic waves with TE and TM polarizations for four thickness values corresponding to the set of the highlighted thickness values in figure 7A: $T_1$ = 2.19 mm, $T_1$ = 2.54 mm, $T_1$ = 3.02 mm and $T_1$ = 3.26 mm. Similar to the phantom device in figure 5C, the phantom device in figure 7C, in all its configurations, provides the angular response similar to that of the reference object with an acceptable tolerance provided for at least one polarization and a certain angular range that can be defined for a given value of the tolerance threshold value.

In an embodiment, the second layer 16 is at least partly transparent to the electromagnetic wave incident on the phantom structure and transmitted through the first dielectric layer 12.

In an embodiment, the second layer 16 is made of a dielectric material having a complex permittivity $\varepsilon_2^*$ and thickness $T_2$ such that the penetration depth of the electromagnetic wave into the second layer 16 is smaller than the thickness of the at least one second layer 16. The penetration depth is defined by the attenuation factor of the second layer. This design allows to minimize the impact of a possible reflection from the bottom surface 17 of the second layer 16 and thus allows to control the elementary reflection from the bottom surface 14 of the first layer 12 by varying the complex permittivity of the second layer 16.

In an advantageous embodiment, the device comprises a first layer 12, a second layer 16 and a third layer. The second layer 16 is made of a dielectric material leading to penetration depth larger than the thickness $T_2$. The second layer 16 is on the third layer and the bottom surface of the second layer 16 is in contact with the top surface of the third layer. The third layer is made of an absorbing material capable of suppressing, at least partly, the back reflection that, otherwise, may alter the amplitude and/or phase of the second reflected wave $E_r^{(s_2)}$ from the bottom surface of the second layer 16. Thus, in this embodiment, the second layer 16 is sandwiched between the first layer and the third layer, and it is possible to vary the complex permittivity and the thickness of the second layer 16 and/or the complex permittivity and thickness of the third layer to adjust the amplitude and phase of the second wave $E_r^{(s_2)}$.

In yet another embodiment, the phantom device comprises a first dielectric layer 12 and a second layer 16. The second layer 16 is made of a composite medium, comprising a first fraction made of a conductive material having an electric conductivity $\sigma_2$ equal at least $10^2$ s/m and a second fraction made of a dielectric material. The elementary reflection and transmission coefficients from and through the bottom surface 14, associated with the interface between the first layer 12 and the second layer 16 is defined by the volume ratio of the two fractions of the compound media of the second layer 16. The second layer 16 comprises a first fraction of a conductive material, being reflecting and thus essentially opaque for the incident electromagnetic waves, and a second fraction of a dielectric material, being at least partly transparent for the incident electromagnetic waves. In other words, such composite medium can be characterized by an effective complex permittivity defined as a function of the volume ratio of the two fractions. Thus, it is possible to vary the total reflection coefficient $r_p$ and the total transmission coefficient by varying the volume ratio of the two fractions or the surface filling factor (SFF).

In a preferred embodiment, the volume ratio between the first fraction and the second fraction is selected in a range between 10% and 90% to provide a relative contribution of a portion of the EM wave reflected from the bottom surface 14, at the interface between the first layer 12 and the second layer 16, into the total reflection coefficient from the upper surface 13 of the phantom device constituting at least 5%.

The figure 8 represents an embodiment of a unit structure 30 comprising a second layer 16 made of a composite medium. The figure 8 represents a cross-sectional view of the second layer 16 in a horizontal XY plane according to an embodiment. The second layer 16 is made of a conductive material, characterized by the electrical conductivity at least equal to $10^2$ S/m, with a thickness $T_2$ being larger than the penetration depth of the EM wave into said conductive material. The conductive material may be a metal, oxide, semiconductor, carbon, graphite, graphene, or a polymer material doped with microparticles of any the said conductive materials. The second layer 16 comprises a through hole 18 with an arbitrary shape in XY plane. The through hole is filled in with a dielectric medium and forms a transparent zone for the EM wave. The dielectric material may be solid dielectric, gel, liquid, or gas. The through hole 18 is characterized by a contour line with length at least equal to a quarter of the wavelength in the medium of the first layer or in the medium filling the through hole 18. The surface area of the through hole is in a range of 10 to 90 % of the total surface of the unit structure. The through holes may have a circular shape or square shape. In this configuration, the surface filling factor (SFF) corresponds to the ratio of the surface occupied by the through hole to the total surface of the unit structure comprising said through hole.

The figure 9A represents the magnitude of the complex transmission and reflection coefficients of a phantom device of figure 6 versus the surface filling factor (SFF) of the conductive inclusions in the medium of the second layer 16. The first layer 12 is made of the same material as in figure 7 characterized by $\varepsilon_1^* = 10.03 - j1.3$ and a thickness $T_1 = 1.55$ mm. The second layer 16 is made of a composite material with a variable surface filling factor (SFF) of conductive inclusions arranged with a regular pattern in a dielectric medium. The figure 9B illustrates schematically the four types of the inclusions referred hereafter as: circular patch, rectangular patch, circular through hole and rectangular through hole. The figure 9A shows curves representing the transmission and reflection coefficients for the four type of inclusions. The figure 9A shows that the EM reflection coefficient can be monotonically tuned from the minimum value of $|r_p| \approx 0.4$ nearly equal to that of the single layer phantom of figure 3 to the maximum value of $|r_p| \approx 0.8$ corresponding to the two-layer structure of figure 6. Thus, by introducing the inclusions, the reflection coefficient is gradually increasing. Depending on the type of the inclusion, the target reference value of $|r_p| \approx 0.61$ is provided for different values of the surface filling factor (SFF) in the range between 35% to 60%. which corresponds to the total transmission coefficient of the device $|t_p|$ in the range between 0.1 to 0.3. Thus, thanks to the device of the present disclosure, the signal-to-noise ratio can be improved for the sensor placed below the phantom layer, on the side opposite to the electromagnetic source 2. This is one of the advantages of the proposed device for electromagnetic dosimetry versus the prior art solutions that aim at reproducing the electromagnetic response by directly reproducing electromagnetic properties of the highly lossy skin tissue.

The figures 12A,12B and 12C illustrate three embodiments of the second composite layer when the phantom device comprises a plurality of unit structures.

The figure 12A represents an example of phantom device comprising four identical unit structures. Each unit structure comprises a first dielectric layer and a second composite layer 16.1, 16.2, 16.3, 16.4. The figure 12A represents a cross-sectional view of the second layers in a horizontal XY plane used in such assembly of four unit structures. Each second layer 16.1, 16.2, 16.3, 16.4 comprises respectively a circular through hole 18.1, 18.2, 18.3, 18.4 filled in with a host medium (such as air) with the surface ratio of about 20% of the surface of the unit structure. In this embodiment, the through holes form isolated objects.

The figure 12B represents an example of phantom device comprising four unit structures. Each unit structure comprises a first dielectric layer and a second composite layer 16.1, 16.2, 16.3, 16.4. The figure 12B represents a cross-sectional view of the second layers in a horizontal XY plane used in such assembly of four unit structures. Each second layer 16.1, 16.2, 16.3, 16.4 comprises respectively a circular through hole 18.1, 18.2, 18.3, 18.4 with the surface ratio of about 80% of the surface of the unit structure. In this embodiment, the through holes merge to form isolated metal object.

The figure 12C represents an example of phantom device comprising four identical unit structures. Each unit structure comprises a first dielectric layer and a second composite layer 16.1, 16.2, 16.3, 16.4. The figure 12C represents a cross-sectional view of the second layers in a horizontal XY plane used in such assembly of four unit structures. Each second layer 16.1, 16.2, 16.3, 16.4 comprises respectively a circular inclusion 18.1, 18.2, 18.3, 18.4 made of a conductive material with the surface ratio of about 20% of the surface of the unit structure. In this embodiment, the circular inclusion form isolated conductive objects.

These two topologies of the through holes result in different electrical and thermal conductivity of the second layer 16. Advantage of the structure in figure 12B with isolated metal patches is that it leads to localized currents and heating, which naturally allows local manipulation over the reflected wave. In figure 12A, the isolated through holes allow local manipulation over the transmitted wave through the second layer 16. The position of the appropriate EM and thermal sensor may be beneficially aligned with either the metal patches of figure 12B or with the holes of figure 12A.

In one embodiment, it can be further beneficial to make the second layer 16 to be at least partly transparent to the electromagnetic wave incident on the top surface of the first layer 12 and transmitted through the first layer 12. This semi-transparent phantom structure can be used in an electromagnetic dosimetry system comprising at least one sensor placed beneath the second layer 16. The sensor may be positioned at a certain distance of the bottoms surface of the second layer 16 or attached to the bottoms surface of the second layer 16. The sensor is configured to measure a physical quantity related to the electromagnetic field incident on the device. The transmission through the structure can be estimated based on the values of the elementary reflection coefficients of the top ($\rho_1$) and bottom ($\rho_2$) surfaces, the thickness $T_1$, and the propagation constant $\gamma_1$ related to the bulk material complex permittivity according to equation (6).

With reference to figure 10, a schematic illustration of a cross-sectional view of another embodiment of a phantom device 40 is described.

The figure 10 represents a reference object 20 in a form of a human head exposed to an electromagnetic wave emitted by a source 2. The reference object 20 comprises an upper surface 21 represented by a set of nodes defined by a topological map. In one approximation, each mesh cell 21.i can be considered as flat and thus characterized by the position of its nodes and the incident angle $\theta_i$, defined with respect to the position of the electromagnetic source that is assumed to be known. Precisely, the incident angle $\theta_i$ is defined relative to the position of the antenna phase center of the electromagnetic source 2 and the surface normal vector. Under this assumption, it is possible to represent each mesh cell by at least one unit structure of the phantom device. Therefore, it is possible to reproduce a part of the surface area of the reference object by a plurality of unit structures. In one embodiment, it can be sufficient to reproduce the EM scattering only from a surface area that is responsible for the back reflection around the electromagnetic source, while all other area that scatter incident electromagnetic waves to other direction than the source, can be neglected. For example, in figure 10, the part of the surface area of the reference object 20 to be reproduced is between A and B. In another embodiment, each unit structure representing a mesh cell of the topological map, is designed to reproduce the magnitude (or both magnitude and phase) of the complex reflection coefficient for the certain angular range of the incident EM wave, defined by the relative position of the source and the surface normal vector associated with each cell. This may allow to reproduce the EM scattering characteristics from a larger surface area.

With reference to figure 10, the phantom device 40 comprises a plurality of unit structures to reproduce the electromagnetic response from a reference object with a complex geometric shape. Each unit structure is characterized by its own complex reflection coefficient $r_{p,n}$, where n is an integer. Each unit structure can be designed as the phantom device of figure 3 or figure 6.

In one example, the unit structure comprises a single dielectric layer 12 as in figure 3. In another example, the unit cell comprises a first dielectric layer 12 and second layer 16 as in figure 6. In yet another further example, at least one unit cell comprises a single dielectric layer 12, a second layer 16 and a third layer. The thickness and/or the effective complex permittivity of a composite material of each layer can be adapted to reproduce locally the electromagnetic response of the reference object.

Thus, the electromagnetic response provided by the plurality of unit structures of the device allows to reproduce the electromagnetic response, both in terms of magnitude and phase from the complex geometric surface 21 of the reference object 20 without reproducing the complex shape of the reference object. For example, the portion of the top surface 41 which reproduces the electromagnetic response from the part (AB) of the surface area of the reference object present a convex shape which is different from that of the part (AB). Thanks to the plurality of unit structures, the device can modify the shape of the top surface different from that of the reference object, while reproducing the electromagnetic response of the reference object.

The figure 11A represents another example of a 3D reference object 90 exposed to a plurality of incident electromagnetic waves 4 from an electromagnetic source 2 incident on the reference object under different incident angles. The reference object 90 presents an external surface having a complex geometric shape.

In one example as illustrated in figure 11B, the phantom device 70 comprises a plurality of unit structures 70.1, 70.2, 70.3, 70.4 which forms a shell in a form resembles to some extent the shape the reference object but is simpler for manufacturing. The unit structures are used to reproduce the realistic EM response from different surface areas of the reference object.

In another example as illustrated in figure 11C, the phantom device 80 comprises a plurality of unit structures 80.1, 80.2, 80.3, 80.4 which forms a planar structure while reproducing locally the electromagnetic response from the corresponding surface areas of the reference object 90.

In one embodiment, the plurality of unit structures is configured to provide a discrete variation of the reflection coefficient. Each unit structure is characterized by its own complex reflection coefficient. The size and the shape of the unit structures can be selected to provide the required variation of the complex reflection coefficient along the surface. When such device is used in an electromagnetic dosimetry, each unit structure may be associated with at least one sensor. For instance, the unit structure may be associated with one single senor, e.g. IR image sensor with a wide field of view measuring the heat.

In another embodiment, the plurality of cell structures is configured to provide a continuous variation of the reflection coefficient with a gradient of the phase and/or amplitude variation along the top surface of the device. The size and the shape of the unit structures can be selected to provide a continuous variation of the reflection coefficient.

In a preferred embodiment, thanks to the use of a plurality of unit structures, it is also possible to adapt the local transparency of the device. Similarly as in case of the reflection from the top surface of the device, each unit structure is characterized by its own transmission coefficient $t_{p,n}$, where n is an integer. In one embodiment, the plurality of cell structures is configured to provide a transmission of the electromagnetic waves through the unit structures in a discrete manner. In another embodiment, the plurality of unit structures is configured to provide a continuous variation of the transmission of the electromagnetic waves through the unit structures. In figure 10, the transparency of the portion of the device between A and B can vary continuously or in a discrete manner.

In one embodiment, the device comprises at least one microelectromechanical switch integrated into at least one unit structure. The switch can change on demand the geometry of the unit structure. For example, the microelectromechanical switch can change the thickness of the unit structure, or the curvature of the top surface of the unit structure. The switch can also be used to control the complex characteristic impedance by varying a size of shape of a parasitic element. The switch can also be used to change on demand the reflection from the second layer 16 made of a composite material by varying the size or shape of the inclusions or spacing between the inclusions.

In one embodiment, the proposed phantom device can wrap a reference object and be attached to the external surface of an object reproducing the shape of a reference object. Thus, the device of the present disclosure which presents initially a planar surface can be conformed into the shape of the object. The device forms thus an artificial skin layer of the object. The artificial skin can reproduce a specific desired value of the complex reflection coefficient of a reference object that can be different from that of the object that is wrapped (or covered) by the phantom device.

In a preferred embodiment, the proposed device can be used to reproduce the complex reflection coefficient of the human tissue, which may be a part of a human body, e.g., head, chest, hand, etc. characterized by a 3D complex shape.

In one embodiment and with reference to figure 13A, the phantom structure 50 is configured to reproduce the electromagnetic response, e.g., the electromagnetic scattering and reflection characteristics of a reference object simultaneously in at least two different frequencies. Most of wireless communication devices can use several frequency bands. For instance, in figure 13A, the source comprises two antennas which can emit the electromagnetic waves at two different frequencies f1, f2 corresponding to different subranged in the frequency range of 6 - 300 GHz.

With reference to figure 13A, the thickness of the first layer 12 is selected in such a way that the device reproduces the two desired values of the complex reflection coefficient at two different frequencies thanks to the difference in phase accumulated by the second wave reflected from the bottom surface 14, defined by the relative thickness of the first layer 12 in term of the wavelength at the corresponding frequency.

In another embodiment and with reference to figure 13B, the thickness of the first layer 12 is selected to be larger than the penetration depth of the EM wave with a frequency f1, while being less than the penetration depth of the EM wave with a frequency f2. Therefore, there is no reflection from the bottom surface 14 at the first frequency f1 and thus there is no contribution to the reflection from the upper surface 13 at the first frequency f1. It results two different EM responses at two different EM response.

In another embodiment and with reference to figure 14, the phantom device 60 further comprises a frequency selective layer 18 having a different reflectance and transmittance at two frequencies. The frequency selective layer 18 can be attached at least to the top surface 13 or to the bottom surface 14. In another embodiment, the frequency selective layer 18 can be embedded in one of the layers.

In figure 14, the phantom device 60 comprises a frequency selective layer 19 embedded in the first layer 12. The frequency selective layer 19 is reflecting for the first incident electromagnetic wave at a first frequency f1 and transparent to the second incident electromagnetic wave at a second frequency f2. In figure 14, the first electromagnetic wave with frequency f1 is reflected from the frequency selective layer 19, while the second electromagnetic wave with frequency f2 is reflected from the bottom surface 14.

In another embodiment, the frequency selective layer 19 comprises an array of resonant elements, such as single or double layer microstrip circuits comprising a resonant stub or patch elements.

The phantom device, thanks to the frequency selective layer, can provide a desired value of the complex reflection coefficient at both frequencies.

With reference to figure 15, a dosimetry system for measuring an electromagnetic quantity according an embodiment of the present disclosure will now be described.

The system 100 comprises a proposed phantom device, for example a phantom device of figure 7 and at least one sensor 101 which is positioned below the phantom device on the side opposite to the location of the electromagnetic source 2. The signal is then transmitted to a signal analyzing unit 101 (SAU) connected to a processing unit 102 (PU) and a memory unit 103 (MU) that are used to post-process and store the measured data.

The sensor 101 is adapted to measure a physical quantity related to the electromagnetic field transmitted through the layer(s) forming the phantom device and forms an electrical signal. The measured physical quantity may be related to the electromagnetic field intensity or the amplitude of the E or H field component of the electromagnetic field transmitted through the different layers of the phantom device. The sensor 101 may be an electromagnetic sensor for measuring the transmitted electromagnetic field, a THz electromagnetic sensor, an infrared electromagnetic sensor, an optical electromagnetic sensor, a thermal sensor, or any other sensor capable of measuring a physical quantity related to the EM field transmitted through the layer(s) of the phantom device.

In one embodiment, the system 100 may further comprise a frequency converting element capable of absorbing the EM field at frequency of the source 2 and reemitting at another frequency, different from that of the source 2. For instance, this second frequency can correspond to the infrared or optical range. In this embodiment, the at least one sensor 101 is an infrared or an optimal image sensor.

**Claims**

1. Phantom device (10) for reproducing at least one electromagnetic (EM) characteristic of a reference object made of an electromagnetic lossy medium, in particular a biological tissue, when illuminated by an EM wave with a predetermined frequency f1 emitted by an EM source (2), said phantom device comprising at least one unit structure, said unit structure comprising:

   - at least one first dielectric layer (12) comprising au upper surface (13) face to the electromagnetic source (2) and a bottom surface (14) opposite to the upper surface (13),
   - said upper surface (13) being at least partly transparent to the electromagnetic waves emitted by the source (2);
   - said bottom surface (14) being at least partly reflecting for the electromagnetic waves transmitted through the first dielectric layer (12);
   - said at least one first dielectric layer (12) **characterized by** a complex dielectric permittivity of its bulk material $\varepsilon_1^* = \varepsilon_1' - j\varepsilon_1''$ having an absolute value in a range between 3 and 40;
   - said at least one first dielectric layer (12) further **characterized by** a thickness $T_1$ at least equal to 1/4 of the wavelength of the EM wave in the medium of the first dielectric layer (12).

2. Phantom device of claim 1, wherein the reference object representing a human tissue, the complex dielectric permittivity $\varepsilon_1^*$ and the thickness $T_1$ of the first dielectric layer (12) are jointly selected so as to reproduce at least the absolute value of the complex reflection coefficient from the surface of the reference object at least for the case of the normal incidence of the EM wave, said absolute value being in the range of 0.40 - 0.75 at the frequency f1 in the frequency range of 6GHz to 300GHz.

3. Phantom device of claim 1 or 2, wherein the at least one first dielectric layer (12) further has the thickness $T_1$ smaller or equal to the penetration depth of the EM wave into the medium of said first dielectric layer (12) and is at least partly transparent to the EM wave at frequency f1 emitted by the EM source (2).

4. Phantom device of any of claims 1 to 3, further comprising at least one second layer (16), the first dielectric layer (12) being positioned on the at least one second layer (16), said at least one second layer (16) being made of a dielectric material, said first dielectric layer (12) and said second dielectric layer (16) jointly configured to reproduce at least the absolute value of the reflection coefficient from the surface of the reference object, with a relative contribution of a portion of the EM wave reflected from the bottom surface (14), at the interface between the first layer (12) and the second layer (16), into the total reflection coefficient from the upper surface (13) of the phantom device constituting at least 5%.

5. Phantom device of claim 4, wherein the second layer (16) has a thickness $T_2$ selected such that the penetration depth of the electromagnetic wave into the medium of the second layer (16) is at least equal to the thickness of said second layer (16), said first dielectric layer (12) and said second dielectric layer (16) being jointly configured to reproduce the absolute value of the complex reflection coefficient from the surface of the reference object, while remaining at least partly transparent to the incident EM wave.

6. Phantom device of any of claims 1 to 5, further comprising at least one second layer (16), the first dielectric layer (12) being positioned on the at least one second layer (16), said at least one second layer (16) being made of a composite medium, comprising a first fraction made of a conductive material having an electric conductivity $\sigma_2$ equal at least $10^2$ s/m and a second fraction made of a dielectric material, the volume ratio between the first fraction and the second fraction being selected in a range between 10% and 90% to provide a relative contribution of a portion of the EM wave reflected from the bottom surface (14), at the interface between the first layer (12) and the second layer (16), into the total reflection coefficient from the upper surface (13) of the phantom device constituting at least 5%.

7. Phantom device of claim 6, wherein the at least one second layer (16) is made of a conductive material, **characterized by** the electrical conductivity at least equal to $10^2$ S/m, with a thickness $T_2$ being larger than the penetration depth of the EM wave into said conductive material, said second layer (16) further comprising at least one through hole (18) forming a transparent zone for the EM wave, said at least one through hole having a surface area in the range of 10 to 90% of the total surface of the unit structure and being filled in with a dielectric medium, said at least one through hole having an arbitrary shape in xy-plane aligned with the bottom surface (14), defined by a contour line with the length at least equal to a half of the wavelength of the EM wave in the dielectric medium filling the at least one through hole (18) or in the medium of the first layer (12).

8. Phantom device of any of claim 4 to 7, wherein the first layer (12) and the second layer (16) are jointly configured to reproduce the at least two different values of the at least one EM characteristic of a reference object when illuminated by two different EM waves, a first EM wave with a frequency f1 within a first frequency subrange and a second EM wave with a frequency f2 within a second frequency sub-range, said two sub-ranges within the frequency range of 6GHz to 300GHz.

9. Phantom device of claim 8, wherein the thickness of the first dielectric layer (12) is smaller than the penetration depth of the first EM wave with the frequency f1, and simultaneously being at least equal to the penetration depth of the second EM wave with the frequency f2.

10. Phantom device of any of claims 4 to 9, further comprising a frequency selective layer (19) being reflecting for a first incident electromagnetic wave at a first frequency f1 and being transparent to a second incident electromagnetic wave at a second frequency f2, the frequency selective layer (19) being embedded in the at least one first layer (12) or attached to the bottom surface (14) of the at least one first layer (12).

11. Phantom device of any of claims 1 to 10, comprising a plurality of unit structures for reproducing locally variable electromagnetic response of the reference object, each unit structure being configured to reproduce the at least one electromagnetic characteristic from a portion of the surface of the reference object.

12. Phantom device of claim 11, wherein the size, the shape and the composition of the layers forming each unit structure, and/or the distance between two adjacent unit structures are selected to obtain a continuous variation of the electromagnetic response along a surface of the phantom system.

13. Phantom device of claim 11, wherein the size, the shape and the composition of the layers forming each unit structure, and/or the distance between two adjacent unit cells are selected to obtain a discrete variation of the electromagnetic response along a surface of the phantom system.

14. Phantom device of any of claims 11 to 13, wherein each unit structure comprises a microelectromechanical switch configured to change the total thickness of the unit structure and/or the curvature of the upper surface of the unit structure, or an effective complex permittivity of the first layer (12) or of the second layer (16).

15. Dosimetry system (100) for measuring an electromagnetic dosimetry quantity related to an electromagnetic field emitted by an electromagnetic source (2), the dosimetry system comprising:

   - a phantom device of any of claims 1 to 14, comprising an upper surface (13) face to the electromagnetic source (2) and a bottom surface (17), said phantom device being at least partly transparent to the EM wave emitted by the electromagnetic source (2);
   - at least one sensor (101) attached to or arranged beneath the bottom surface (17) and configured to measure a physical quantity related to the electromagnetic wave transmitted through the phantom device;
   - a signal analyzing unit (102) configured to analyze the signal transmitted from the at least one sensor (101), a processing unit (103) configured to calculate the electromagnetic dosimetry quantities from the signal and a memory unit (104).

16. Dosimetry system of claim 15, wherein the at least one sensor (101) comprises an electromagnetic sensor operating at a frequency of the electromagnetic source (2).

17. Dosimetry system of claim 15, wherein the at least one sensor (101) comprises an electromagnetic sensor operating at a frequency different from that of the electromagnetic source (2), the device further comprising a frequency converter element.

18. Dosimetry system of claim 15, wherein the at least one sensor (101) comprises a thermal sensor.

FIG. 1

FIG. 2A

**FIG. 2B**

**FIG. 2C**

**FIG. 3**

**FIG. 4**

**FIG. 5A**

**FIG. 5B**

**FIG. 5C**

## FIG. 6

## FIG. 7A

## FIG. 7B

**FIG. 7C**

**FIG. 8**

**FIG. 9A**

**FIG. 9B**

**FIG. 10**

**FIG. 11A**

**FIG. 11B**

**FIG. 11C**

**FIG. 12A**

**FIG. 12B**

**FIG. 12C**

$E_i^{(1)}(f_1)$

$E_r^{(1)}(f_1)$

$E_i^{(1)}(f_2)$

$E_r^{(1)}(f_2)$

$E_r^{(2)}(f_1)$

$E_r^{(2)}(f_2)$

$E_t^{(2)}(f_1)$

$E_t^{(2)}(f_2)$

$T_1$

$T_2$

**FIG. 13A**

$E_i^{(1)}(f_1)$

$E_r^{(1)}(f_1)$

$E_i^{(1)}(f_2)$

$E_r^{(1)}(f_2)$

$E_t^{(1)}(f_1)$

$E_r^{(2)}(f_2)$

$E_t^{(2)}(f_2)$

$T_1$

$T_2$

**FIG. 13B**

$E_i^{(1)}(f_1)$  $E_i^{(1)}(f_2)$

$E_r^{(1)}(f_1)$  $E_r^{(1)}(f_2)$

$T_1'$  $T_1$  $T_2$

**FIG. 14**

$E_r^{(1)}$

$E_i^{(1)}$

$E_t^{(1)}$

$E_r^{(2)}$

$E_t^{(2)}$

Layer 1

Layer 2

$T_1$  $T_2$

SAU  PU  MU

**FIG. 15**

Europäisches
Patentamt

European
Patent Office

Office européen
des brevets

## EUROPEAN SEARCH REPORT

Application Number

EP 21 30 6476

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GURALIUC ANDA R ET AL: "Solid Phantom for Body-Centric Propagation Measurements at 60 GHz", IEEE TRANSACTIONS ON MICROWAVE THEORY AND TECHNIQUES, IEEE, USA, vol. 62, no. 6, 1 June 2014 (2014-06-01), pages 1373-1380, XP011549851, ISSN: 0018-9480, DOI: 10.1109/TMTT.2014.2320691 [retrieved on 2014-06-02] | 1-3, 11-13, 15-18 | INV. G01R29/08 G09B23/30 H04B17/00 H04M1/24 |
| A | * page 1373 - page 1378; figures 1-7 * | 4-10,14 | |
| X | ZIANE MASSINISSA ET AL: "High-Resolution Technique for Near-Field Power Density Measurement Accounting for Antenna/Body Coupling at Millimeter Waves", IEEE ANTENNAS AND WIRELESS PROPAGATION LETTERS, IEEE, PISCATAWAY, NJ, US, vol. 20, no. 11, 7 June 2021 (2021-06-07), pages 2151-2155, XP011888269, ISSN: 1536-1225, DOI: 10.1109/LAWP.2021.3087019 [retrieved on 2021-11-15] | 1-3, 11-13, 15-18 | |
| | | | TECHNICAL FIELDS SEARCHED (IPC) |
| A | * page 2151 - page 2154; figures 1-4 * | 4-10,14 | G01R H04B G09D |
| X | GURALIUC ANDA R ET AL: "Effect of Textile on the Propagation Along the Body at 60 GHz", IEEE TRANSACTIONS ON ANTENNAS AND PROPAGATION, IEEE, USA, vol. 62, no. 3, 1 March 2014 (2014-03-01), pages 1489-1494, XP011541769, ISSN: 0018-926X, DOI: 10.1109/TAP.2013.2295425 [retrieved on 2014-02-27] | 1-3, 11-13, 15-18 | H04M G09B |
| A | * page 1489 - page 1492; figures 1-7 * | 4-10,14 | |

-/--

The present search report has been drawn up for all claims

| Place of search | Date of completion of the search | Examiner |
|---|---|---|
| Munich | 7 April 2022 | Bilzer, Claus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 21 30 6476

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 326 070 A1 (NTT DOCOMO INC [JP]) 9 July 2003 (2003-07-09) | 1-3, 11-13, 15-18 | |
| A | * paragraphs [0015] - [0058]; claims 1-20; figures 1-21 * | 4-10,14 | |
| X | TAKEHIKO KOBAYASHI ET AL: "DRY PHANTOM COMPOSED OF CERAMICS AND ITS APPLICATION TO SAR ESTIMATION", IEEE TRANSACTIONS ON MICROWAVE THEORY AND TECHNIQUES, IEEE, USA, vol. 41, no. 1, 1 January 1993 (1993-01-01), pages 136-140, XP000358558, ISSN: 0018-9480, DOI: 10.1109/22.210240 | 1-3, 11-13, 15-18 | |
| A | * page 136 - page 139; figures 1-7 * | 4-10,14 | |
| A | PELLEGRINI A ET AL: "Antennas and Propagation for Body-Centric Wireless Communications at Millimeter-Wave Frequencies: A Review [Wireless Corner]", IEEE ANTENNAS AND PROPAGATION MAGAZINE, IEEE SERVICE CENTER, PISCATAWAY, NJ, US, vol. 55, no. 4, 1 August 2013 (2013-08-01), pages 262-287, XP011531255, ISSN: 1045-9243, DOI: 10.1109/MAP.2013.6645205 [retrieved on 2013-10-22] * page 262 - page 270; figures 1-10 * | 1-18 | TECHNICAL FIELDS SEARCHED (IPC) |

-/--

The present search report has been drawn up for all claims

| Place of search | Date of completion of the search | Examiner |
|---|---|---|
| Munich | 7 April 2022 | Bilzer, Claus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

**EP 21 30 6476**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GABRIEL C: "Tissue equivalent material for hand phantoms", PHYSICS IN MEDICINE AND BIOLOGY, INSTITUTE OF PHYSICS PUBLISHING, BRISTOL GB, vol. 52, no. 14, 21 July 2007 (2007-07-21), pages 4205-4210, XP020112970, ISSN: 0031-9155, DOI: 10.1088/0031-9155/52/14/012 * page 4205 – page 4209; figures 1-3 * | 1-18 | |
| A | NACER CHAHAT ET AL: "Broadband Tissue-Equivalent Phantom for BAN Applications at Millimeter Waves", IEEE TRANSACTIONS ON MICROWAVE THEORY AND TECHNIQUES, IEEE, USA, vol. 60, no. 7, 1 July 2012 (2012-07-01), pages 2259-2266, XP011448665, ISSN: 0018-9480, DOI: 10.1109/TMTT.2012.2195196 * page 2259 – page 2263; figures 1-8 * | 1-18 | |

TECHNICAL FIELDS
SEARCHED      (IPC)

The present search report has been drawn up for all claims

| Place of search | Date of completion of the search | Examiner |
|---|---|---|
| Munich | 7 April 2022 | Bilzer, Claus |

CATEGORY OF CITED DOCUMENTS

X : particularly relevant if taken alone
Y : particularly relevant if combined with another
      document of the same category
A : technological background
O : non-written disclosure
P : intermediate document

T : theory or principle underlying the invention
E : earlier patent document, but published on, or
      after the filing date
D : document cited in the application
L : document cited for other reasons
 
& : member of the same patent family, corresponding
      document

EPO FORM 1503 03.82 (P04C01)

## ANNEX TO THE EUROPEAN SEARCH REPORT
## ON EUROPEAN PATENT APPLICATION NO.

EP 21 30 6476

This annex lists the patent family members relating to the patent documents cited in the above-mentioned European search report.
The members are as contained in the European Patent Office EDP file on
The European Patent Office is in no way liable for these particulars which are merely given for the purpose of information.

07-04-2022

| Patent document cited in search report | | Publication date | Patent family member(s) | | Publication date |
|---|---|---|---|---|---|
| EP 1326070 | A1 | 09-07-2003 | CA | 2421821 A1 | 07-03-2003 |
| | | | CN | 1473268 A | 04-02-2004 |
| | | | CN | 1651907 A | 10-08-2005 |
| | | | EP | 1326070 A1 | 09-07-2003 |
| | | | EP | 2199811 A1 | 23-06-2010 |
| | | | JP | 3809166 B2 | 16-08-2006 |
| | | | JP | WO2003014717 A1 | 25-11-2004 |
| | | | US | 2004102694 A1 | 27-05-2004 |
| | | | US | 2006172432 A1 | 03-08-2006 |
| | | | WO | 03014717 A1 | 20-02-2003 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 20170173350 A